(19) Europäisches Patentamt
European Patent Office
Office européen des brevets

(11) Veröffentlichungsnummer: **0 591 807 A2**

(12) # EUROPÄISCHE PATENTANMELDUNG

(21) Anmeldenummer: **93115564.2**

(22) Anmeldetag: **27.09.93**

(51) Int. Cl.$^5$: **C08F 8/00**

(30) Priorität: **09.10.92 DE 4234079**
**09.07.93 DE 4322884**

(43) Veröffentlichungstag der Anmeldung:
**13.04.94 Patentblatt 94/15**

(84) Benannte Vertragsstaaten:
**BE CH DE DK ES FR GB IT LI NL SE**

(71) Anmelder: **BAYER AG**

**D-51368 Leverkusen(DE)**

(72) Erfinder: **Heiliger, Ludger Dr.**
**Carl- Rumpff-Strasse 8**
**D-51373 Leverkusen(DE)**
Erfinder: **Kuckert, Eberhard Dr.**
**Am Scherfenbrand 67**
**D-51375 Leverkusen(DE)**
Erfinder: **Löbberding, Antonius Dr.**
**Am Rohm 105**
**D-42113 Wuppertal(DE)**
Erfinder: **Springer, Wolfgang, Dr.**
**Katernberger Schulweg 31**
**D-42113 Wuppertal(DE)**

(54) **Biologisch aktive Polymere.**

(57) Die vorliegende Erfindung betrifft neuartige, biologisch aktive Polymere, Verfahren zu deren Herstellung sowie ihre Verwendung für biochemische Erkennungsreaktionen und/oder zur weiteren Umsetzung mit biologisch aktiven Molekülen, wie beispielsweise Proteinen oder Nukleinsäuren.

**EP 0 591 807 A2**

Die vorliegende Erfindung betrifft neuartige, biologisch aktive Polymere, Verfahren zu deren Herstellung sowie ihre Verwendung für biochemische Erkennungsreaktionen und/oder zur weiteren Umsetzung mit biologisch aktiven Molekülen, wie beispielsweise Proteinen oder Nukleinsäuren.

In der WO 89/053 29 werden Polymeroberflächen von fertigen, an sich wasserunlöslichen Polymeren, wie Mikrotitrierplättchen aus Polystyrol oder Polymethylmethacrylat mittels photochemisch aktiven, biotinylierten Verbindungen in wässriger Lösung anhand polymeranaloger Photoreaktion modifiziert. Die photochemische Verknüpfung der photochemisch reaktiven monomeren Verbindung mit dem Polymeren geschieht in heterogener Phase mit dem von der Lösung benetzten Teil des Polymeren, d.h. nur an den sich berührenden Oberflächen der beiden Phasen. Die hohe Oberflächenspannung des Wassers, die sich in der schlechten Benetzbarkeit des Kunststoffplättchens niederschlägt, sorgt zudem für einen extrem schlechten Kontakt beider Komponenten, die zur Reaktion gelangen sollen. Daraus ergibt sich eine schlechte Quantenausbeute der Photoverknüpfungsreaktion und eine lange Bestrahlungszeit (1,5 h), um überhaupt Verknüpfungsreaktionen erfolgreich durchführen zu können. Die so statistisch funktionalisierte, feste Polymeroberfläche läßt sich prinzipiell nur noch für die Immobilisierung von Biomolekülen über die an sich bekannte Biotin - Avidin - (oder Streptavidin -) Biotin -Biomolekül Sequenz erreichen. Dazu ist es notwendig, auch das zu immobilisierende Biomolekül zuerst einer Funktionalisierung mit Biotin zu unterwerfen. Dabei ist die eigentliche Immobilisierung eines biotinylierten Biomoleküls nur in einem extrem engen Konzentrationsbereich experimentell zugänglich, weil (Strept-) Avidin, als mehrfachfunktionelles Reagenz gegenüber Biotin, gleichermaßen als Brückenglied in der Verknüpfungssequenz, wie auch als Blockiersubstanz (d.h. Vernetzung zwischen den einzelnen Polymermolekülen der Titrierplättchen) reagieren kann. Das bedeutet, daß bei niedriger Konzentration an Biotin-Ankergruppen eine Verknüpfung mit (biotinylierten) Biomolekülen über (Strept-) Avidin nur in geringen Ausbeuten verlaufen kann, während bei hohen Konzentrationen von Biotin-Ankergruppen eine Totalvernetzung mit dem Mikrotitrierplättchen und damit keine Verknüpfung mit (biotinylierten) Biomolekülen mehr erfolgt.

Erfindungsgemäße Polymere lassen sich dagegen in homogener Phase mit Biomolekülen, wie Nukleinsäuren oder Proteinen verknüpfen und somit nicht nur für eine direkte, spezifische Immobilisierung, sondern darüberhinaus auch über die spezifische biochemische Erkennungsreaktion zur Quantifizierung von Biomolekülen einsetzen. Durch den gezielten Einbau des biologisch aktiven Teils, tritt auch bei hohem Umsatz einer Verknüpfungsreaktion keine Vernetzung auf.

Es wurden nun biologisch aktive Polymere der allgemeinen Formel (I) gefunden

P-(A)q (I)

in welcher

- P eine polymere Komponente,
- A ein biologisch aktiver Teil und
- q die Zahl 1 oder 2 bedeutet.

Als biologisch aktiver Teil A kommen beispielsweise Biotin, Digoxigenin, Digoxin, Digitoxigenin, Digitoxin und Oligonukleotide aus 1 bis 80, vorzugsweise 15 bis 50 und insbesondere 20 bis 35 Nukleotidbausteinen, in Frage. Biotin, Digoxigenin und Oligonukleotid mit 15 bis 50, insbesondere 20 bis 35 Nukleotidbausteinen sind bevorzugt.

Die polymere Komponente P kann linear, verzweigt oder vernetzt sein und läßt sich beispielsweise durch radikalische Polymerisation oder Polykondensation herstellen. Polyurethane und Polyharnstoffe sind ebenfalls geeignet.

Als Monomerbausteine der polymeren Komponente kommen beispielsweise Acryl-, Methacryl-, Vinyl- oder Styryl-Derivate oder Mischungen davon in Frage. Dabei kann es sich beispielsweise um deren Säure-, Ester-, Amid- oder Keton-Derivate handeln.

Als Monomerbausteine sind bevorzugt Verbindungen der Formel (II) geeignet

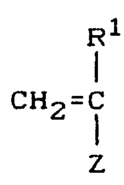

(II)

in welcher

Z für Wasserstoff, $C_1$-$C_{20}$-Alkyl, vorzugsweise $C_1$-$C_{15}$-Alkyl, insbesondere $C_1$-$C_6$-Alkyl, -CO-$OR^2$, -CO-$NR^3R^4$ oder -$OOCR^5$ steht und

$R^1$, $R^2$, $R^3$, $R^4$ und $R^5$ unabhängig voneinander Wasserstoff oder $C_1$-$C_{20}$-Alkyl, vorzugsweise $C_1$-$C_{15}$-Alkyl, insbesondere $C_1$-$C_6$-Alkyl, bedeuten.

Als weitere monomere Bausteine kommen bevorzugt Styrol, α-Methylstyrol oder Verbindungen der Formel (III) in Frage

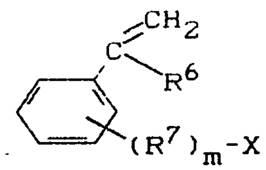

(III)

in der

$R^6$ Wasserstoff oder Methyl,

$R^7$ $CH_2$ oder $SO_2$,

m Null oder 1 und

X Halogen, $SO_2$-$CH_2$-$CH_2$-Halogen, OMe, SO-$CH_3$ oder Methyl

bedeuten.

Halogen steht dabei vorzugsweise für Chlor, Brom oder Iod, insbesondere Chlor oder Brom, und Me für ein Äquivalent eines Metalls, beispielsweise Natrium, Kalium, Caesium oder Ammonium.

Ebenso kommen 1- und 2-Vinylnaphthalin, 1-Vinylcarbazol und Verbindungen in Frage, die zu solchen der Formel (III) analog sind, als aromatischen Grundkörper jedoch Naphthalin oder Carbazol enthalten, sowie sich von aromatischen Aminen, Phenolen, aromatischen Hydroxycarbon-, Hydroxysulfon-, Aminocarbon- und Aminosulfonsäuren ableitenden (Meth)Acrylamide und (Meth)Acrylate der Formel (IV)

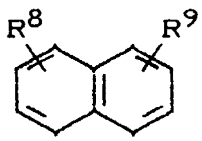

(IV)

in der

$R^8$ für Wasserstoff, $SO_3H$, COOH, $SO_3Me$ oder COOMe steht, wobei Me ein Äquivalent eines Metalls wie beispielsweise Natrium, Kalium, Caesium oder Ammonium ist, bedeutet und

$R^9$ für

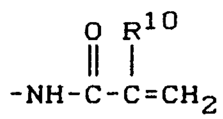

oder

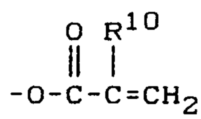

mit $R^{10}$ = Wasserstoff oder Methyl

steht.

Die Polykondensate, Polyurethane oder Polyharnstoffe sind bekannt, deren allgemeine Herstellungsverfahren werden in Houben-Weyl, Makromolekulare Chemie, Teil 1, (1987), S. 555-608 beschrieben. Zusätzlich sind im Teil 2, S. 1443-1457 und 1561-1751 eine große Anzahl dieser Polymere detailliert für verschiedene Anwendungszwecke näher beschrieben.

Die oben genannten Monomerbausteine können untereinander in Form von statistischen, alternierenden oder verzweigten Block-, Pfropf- oder Kammpolymeren kombiniert werden. Bevorzugt sind statistische Copolymere sowie Homopolymere.

Nichtvernetzte, nichtionische Polymere P haben mittlere Molekulargewichte ($\overline{M}_w$) zwischen 1 000 und 10 000 000, bevorzugt zwischen 5 000 und 2 000 000. Polykondensationsprodukte haben vorzugsweise mittlere Molekulargewichte zwischen 5 000 und 100 000.

Ionische Polymere haben in der Regel eine Grenzviskosität von mindestens 0,1 dl/g, bevorzugt 0,5 bis 20, insbesondere 1-10 dl/g gemessen in 0,9 % iger wäßriger NaCl-Lösung bei 20 ° C.

Die Monomerbausteine können reaktive oder aktivierbare Gruppen enthalten, welche die kovalente Bindung beispielsweise zu einem Chelatbildner ermöglichen. Derartige Gruppen können beispielsweise Säurehalogenid-, Imidester-, Benztriazolyl-, Isocyanato-, Isothiocyanato-, Oxiran oder Diimid-Gruppen sein. Reaktive Gruppen tragende Monomerbausteine sind beispielsweise (Meth)Acrylsäurechlorid, (Meth)-Acrylsäure-N-hydroxy-succinimidester, (Meth)Acrylsäure-N-hydroxy-phthalimidester, N-(Meth)-acryloylbenztriazol, 3- oder 4-Isothiocyanatophenyl-(meth)acrylat,2-Isocyanatoethyl(meth)acrylat, Isocyanatoisopropenyl-benzol, Isopropenyl-$\alpha,\alpha$-dimethylbenzylisocyanat, Vinyloxiran oder eine Kombination aus (Meth)Acrylsäure mit Carbodiimiden genannt.

Als Chelatbildner im Sinne der Erfindung werden alle Strukturen verstanden, die in der Lage sind, ein-, zwei- und dreiwertige Metallionen reversibel zu komplexieren.

Mögliche Chelatbildner sind beispielsweise

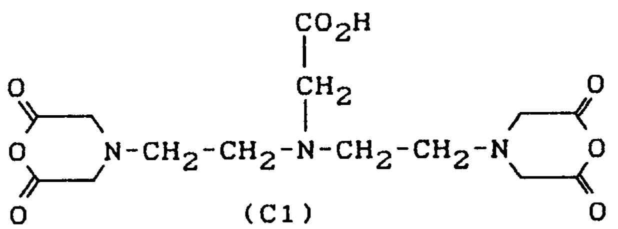

(C1)

oder

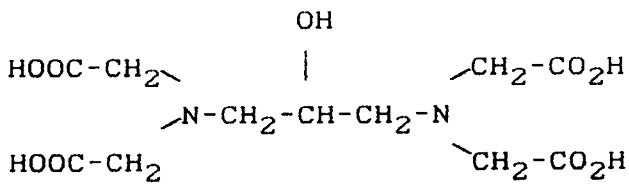

(C2)

(z.T. nach Öffnung cyclischer Säureanhydridfunktionen).

Als Monomerbausteine mit chelatbildenden Gruppen sind beispielsweise die folgenden Verbindungen genannt:

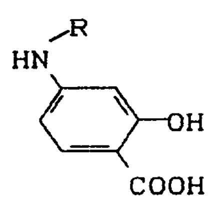

(C3)

worin

R = 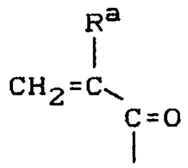 oder 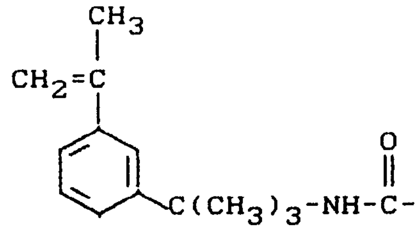

mit $R^a$ = H, $CH_3$

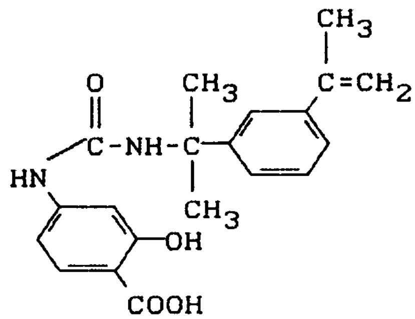

(C4)

mit R = H, $CH_3$

Eine Vielzahl von geeigneten Chelatbildnern sind beschrieben bei L. Yuanfang und W. Chuanchu, Pure and Applied Chemistry, Vol. 63 No. 3, 427-463 (1991).

Falls im biologisch aktiven Polymer chelatbildende Gruppen enthalten sind, beträgt der Anteil der Monomerbausteine mit chelatbildenden Gruppen vorzugsweise 10-90, insbesondere 30-80 Gew.% bezogen auf 100 Gew.% Gesamt-Polymer.

Die Monomerbausteine der Komponente P können auch wasserlöslich machende ionische oder nichtionische Gruppen enthalten. Vorzugsweise enthalten die Monomere ionische wasserlöslich machende Gruppen.

Bevorzugte Monomerbausteine enthalten wasserlöslichkeitsvermittelnde Gruppen, um so dem Polymer Wasserlöslichkeit zu vermitteln, die für die weitere Verknüpfung mit biologisch aktiven Substraten vorteilhaft ist. Von den wasserlöslichkeitsvermittelnden Gruppen sind Carbon- und Sulfonsäure-Gruppen sowie deren konjugierte Basen besonders bevorzugt. Besonders bevorzugt sind polymere Komponenten P, die für ein-, zwei und drei-wertige Kationen, wie beispielsweise Lanthaniden, insbesondere Europium, chelatisierend wirken.

Es kann sich dabei beispielsweise um p-Styrylsulfonsäuresalz ($Na^+$,$K^+$, $NH_4$), Acrylsäure, Methacrylsäure, Acrylamid, Methacrylamid oder Derivate davon handeln. Als Derivate kommen z.B. in Frage: 2-Acryloylamino-2-methyl-propansulfonsäure, Dialkylamino-alkyl-(meth)acrylate und Dialkylamino-alkyl-(meth)-acrylamide wie Dimethylaminoethyl-methacrylat, Dimethylamino-propylacrylamid und die sich von solchen (Meth)acrylaten und (Meth)acrylamiden ableitenden quarternierten Verbindungen. Ferner kommen beispielsweise in Frage: N-Vinylpyrrolidon, N-Vinylpiperidon, N-Vinylcaprolactam, N-Vinylformamid, N-Vinylacetamid, N-Vinyl-N-methyl-acetamid und N-Vinyl-O-methylurethan.

Die Menge an Monomerbausteinen mit wasserlöslich machenden Gruppen im Gesamtpolymer beträgt vorzugsweise 10 bis 90 Gew.% insbesondere 20 bis 70 Gew.% bezogen auf 100 Gew.-% Gesamtpolymer.

Die Komponente P kann neben chelatisierenden und/oder wasserlöslich machenden Gruppen Farbstoffe, insbesondere Fluoreszenzfarbstoffe, enthalten. Vorzugsweise enthält die Polymerkomponente Farbstoffe.

Als Fluoreszenzfarbstoffe kommen beispielsweise in Frage Cumarine der Formel (Va)

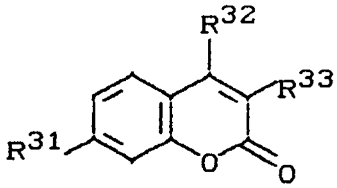

(Va),

in der

$R^{31}$ für O-Alkyl, $N(Alkyl)_2$, NH-Alkyl, $NH-SO_2$-Alkyl, O-Trimethylsilyl oder $NH-SO_2$-Aryl,

$R^{32}$ für Wasserstoff, Cyano, Chlor, Hydroxy, Alkyl oder Aryl und

$R^{33}$ für Phenyl oder Hetaryl stehen.

Alkyl bedeutet dabei vorzugsweise $C_1$- bis $C_6$-Alkyl, Aryl vorzugsweise Phenyl, Alkylen vorzugsweise $C_1$-$C_6$-Alkylenund Hetaryl vorzugsweise (Benz)thiazolyl

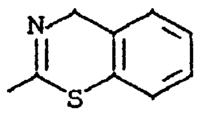

,

$R^{31}$ kann außerdem

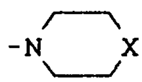

bedeuten, wobei X für Sauerstoff, $N-C_1$-

$C_4$-Alkyl oder $(CH_2)_n$ steht, worin n = 0 oder 1 sein kann.

Weiterhin geeignet sind Cumarine der Formel (Vb)

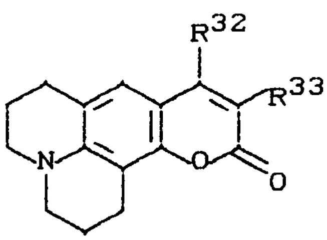

(Vb),

worin

$R^{32}$ und $R^{33}$ die bei Formel (Va) angegebene Bedeutung haben.

Vorzugsweise enthalten die Cumarine der Formeln (Va) und (Vb) an einem der Substituenten $R^{31}$, $R^{32}$ und $R^{33}$ eine funktionelle Gruppe für die Verknüpfung des Farbstoffs mit einem Monomerbaustein oder dem daraus zugänglichen Polymer. $NH_2$- oder OH-Gruppen sind dafür besonders geeignet.

Ebenfalls geeignet sind Carbostyrile der Formel (Vc)

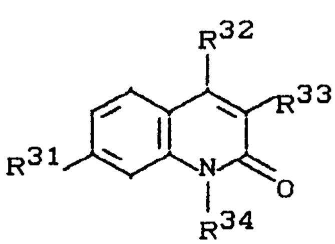

(Vc),

worin

| | |
|---|---|
| $R^{31}$, $R^{32}$ und $R^{33}$ | die bei den Cumarinen (siehe Formeln (Va) und (Vb) angegebenen Bedeutungen haben und |
| $R^{34}$ | für Alkyl, vorzugsweise für $C_1$- bis $C_6$-Alkyl steht. |

Auch hier enthält einer der Substituenten vorzugsweise eine funktionelle Gruppe für die Verknüpfung mit einem Monomerbaustein oder dem daraus zugänglichen Polymer.

Weiterhin sind Pyrazoline der Formel (Vd) geeignet

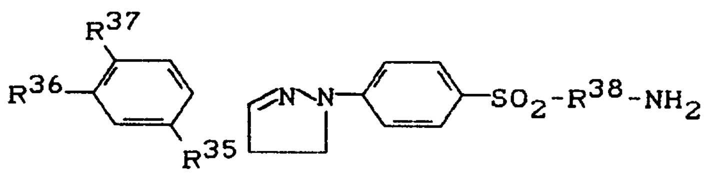

(Vd),

worin

| | |
|---|---|
| $R^{35}$ | für Wasserstoff oder Methyl, |
| $R^{36}$ und $R^{37}$ | unabhängig voneinander für Wasserstoff oder Chlor und |
| $R^{38}$ | für Alkylen, |

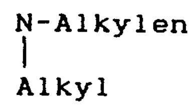

oder Alkylen-O-Alkylen stehen,

wobei Alkyl und Alkylen beispielsweise $C_1$- bis $C_6$-Alkyl oder $C_1$- bis $C_6$-Alkylen bedeuten kann.

Auch geeignet sind Naphthalimide der Formel (Ve)

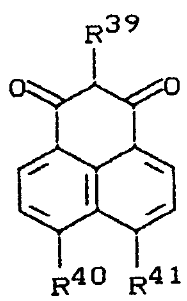

(Ve),

worin

| | |
|---|---|
| $R^{39}$ | für Alkyl und |
| $R^{40}$ und $R^{41}$ | unabhängig voneinander für Wasserstoff, O-Alkyl oder $N(Alkyl)_2$ stehen, |

wobei Alkyl vorzugsweise jeweils $C_1$- bis $C_6$-Alkyl bedeutet und einer der Reste $R^{39}$, $R^{40}$ oder $R^{41}$ eine $NH_2$-Gruppe zur Verknüpfung mit einem Monomerbaustein oder dem daraus zugänglichen Polymer trägt.

Ebenfalls geeignet sind Pyrene der Formel (Vf)

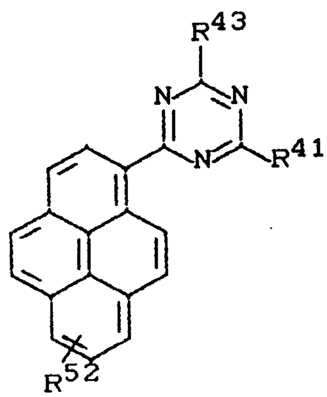

(Vf),

worin

$R^{42}$ für Wasserstoff oder $SO_3H$ und

$R^{43}$ und $R^{44}$ unabhängig voneinander für O-Alkyl oder $N(Alkyl)_2$ stehen,

wobei Alkyl vorzugsweise $C_1$- bis $C_6$-Alkyl bedeutet und einer der Reste $R^{43}$ oder $R^{44}$ eine $NH_2$-Gruppe zur Verknüpfung mit einem Monomerbaustein oder dem daraus zugänglichen Polymer trägt.

Es kommen auch Fluoresceine der Formel (Vg)

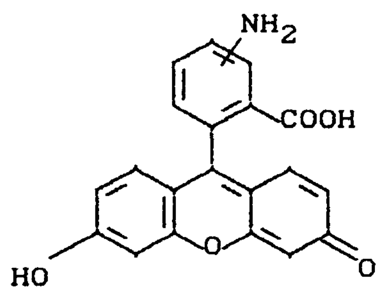

(Vg),

und Rhodamine der Formel (Vh) in Frage

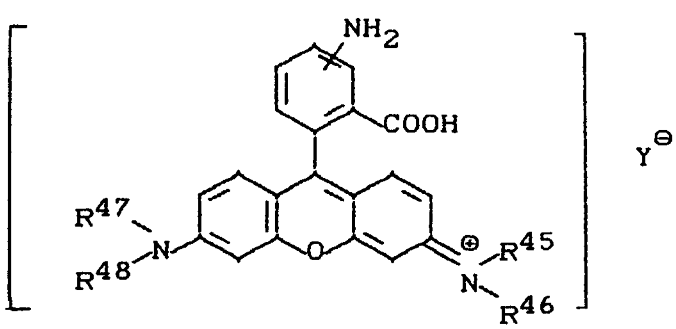

(Vh),

worin

$Y^{\ominus}$ ein farbloses Anion, z.B. $Cl^{\ominus}$, $Br^{\ominus}$, $I^{\ominus}$, $HSO_4^{\ominus}$,

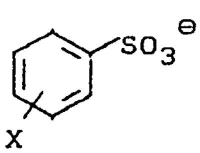

X = Cl, Br, I, $CH_3$

bedeutet und

$R^{45}$ bis $R^{48}$ unabhängig voneinander für Alkyl oder

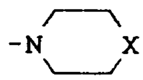

stehen,
wobei Alkyl vorzugsweise $C_1$- bis $C_6$-Alkyl bedeutet und X für Sauerstoff, N-$C_1$- bis $C_4$-Alkyl oder $(CH_2)n$ steht, worin n Null oder 1 sein kann.

$\overset{\oplus}{N}R^{45}R^{46}$ und/oder $NR^{47}R^{48}$ können auch zusammen mit dem aromatischen Ring, an den sie gebunden sind, ein polycyclisches System bilden, z.B. ein System der Formel (Vi) oder (Vk).

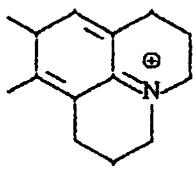 (Vi) 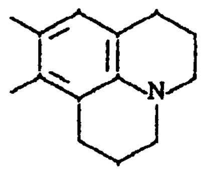 (Vk)

Diese und weitere geeignete Farbstoffe sind bekannt (siehe z.B. "The Chemistry of Synthetic Dyes", Vol. V, Academic Press (1971) und "Fluorescent Whitening Agents", G. Thieme Verlag Stuttgart (1975)).

Bevorzugt sind Cumarine, Fluorescine und Rhodamine.

Bevorzugt enthält die Komponente P 1 bis 99 Gew.-%, insbesondere 5 bis 50 und ganz besonders bevorzugt 10 bis 40 Gew.-% Farbstoff enthaltende Monomerbausteine, bezogen auf das Gesamtpolymer. Der restliche Monomer-Anteil der Komponente P enthält vorzugsweise wasserlöslich machende Gruppen.

Im einzelnen seien beispielsweise die folgenden erfindungsgemäßen Polymere der Formel (I) genannt
Biotin-Polymer 1
Biotin-Polymer 2
Biotin-Polymer 3
Biotin-Polymer 4
Biotin-Polymer 5
Oligionukleotid-Polymer 5
Monomereinheit des Polymer 1 mit chelatbildender Gruppe:

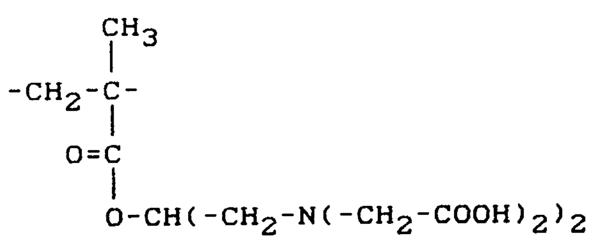

Monomereinheit des Polymer 2 mit chelatbildender Gruppe:

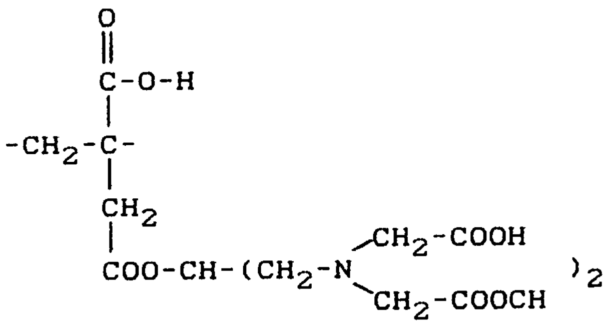

Momomereinheit des Polymer 3, mit chelatbildender Gruppe:

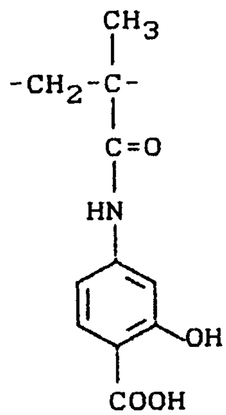

Monomereinheit des Polymer 4 mit chelatbildender Gruppe:

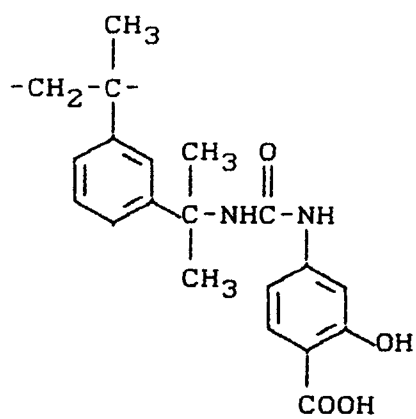

Monomereinheit des Polymer 5:

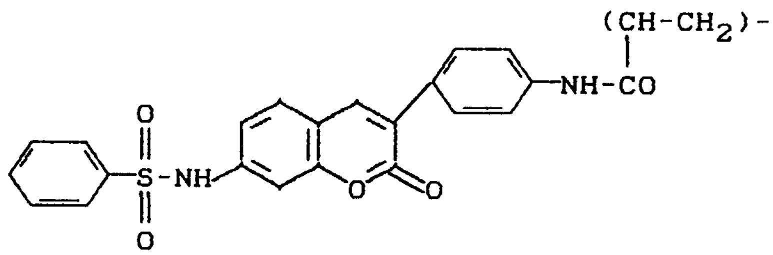

und

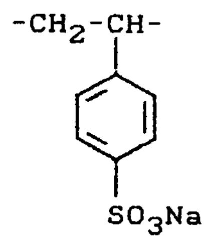

Zusätzlich können erfindungsgemäße biologisch verknüpfbare Polymere noch durch die Herstellungsart bedingt Radikalkettenstarter-Reste enthalten, welche den Resten B-L in Formel (VII) entsprechen.

Die Bausteine A und P der erfindungsgemäßen Polymere sind beispielsweise über eine Ester-, Sulfonsäureester-, Amid-, Sulfonamid-, Urethan-, Thiourethan-, Harnstoff-, Thio-Harnstoff-, Ether-, Amin- und Sulfid-Gruppe miteinander verknüpft. Falls eine erhöhte Beweglichkeit der Bausteine A und/oder P erwünscht ist, können zwischen den Verknüpfungsgruppen Reste wie beispielsweise $C_1$-$C_{20}$-Alkylen, vorzugsweise $C_3$-$C_{15}$-Alkylen, insbesondere $C_5$-$C_{10}$-Alkylen, $C_6$-$C_{10}$-Arylen-$C_2$-$C_{10}$-Alkylen, vorzugsweise Phenylen- bzw. Naphthylen-$C_2$-$C_8$-Alkylen, oder $(CH_2$-$CH_2$-$O)_1$ mit 1 = 1-20, vorzugsweise 3-15, insbesondere 5-10, eingebaut sein.

Bevorzugt sind die Bausteine A und P über eine Amid-, Harnstoff-, Ester-, Ether- oder Urethan-Gruppe verknüpft, besonders bevorzugt sind Amid-, Polyether- und Harnstoff-Gruppen.

Die Monomerbausteine sind allgemein bekannt. Die polymere Komponente P läßt sich nach allgemein bekannten Polymerisationsverfahren herstellen.

So werden beispielsweise Vinylmonomere nach den üblichen Bedingungen der radikalischen Polymerisation mit Radikalkettenstartern, die den biologisch aktiven Teil A enthalten (co-) polymerisiert, d.h. bei Temperaturen von 30 bis 150 °C, wenn erwünscht oder bei festen Reaktanten in (inerten) Lösemittel, wie beispielsweise Dimethylacetamid, Dimethylformamid, Dimethylsulfoxid, (chlorierte) Aromaten, (chlorierte) Aliphaten, Ether, Ester, Ketone, Alkohole, bei atmosphärischem Druck oder bei gasförmigen Monomeren auch bei Überdruck in Autoklaven umsetzt. Wenn es gewünscht ist, können noch übliche Zusätze wie ($\overline{M}_w$-Regler wie beispielsweise Dodecanthiol oder n-Butanthiol, Vernetzer bei der Herstellung von Beads wie beispielsbeise Divinylbenzol, etc.) der Reaktionslösung beigesetzt werden.

Die den biologisch aktiven Teil A enthaltenden Radikalkettenstarter (Verbindungen der Formel (VII) s.u.) sind beispielsweise durch Umsetzung von amino-oder hydroxy-funktionellen Verbindungen, die den biologisch reaktiven Teil A enthalten, mit (sulfon)-säurechlorid- oder isocyanat-funktionellen Radikalkettenstartern zugänglich.

Der biologisch aktive Teil A, der in den Polymeren der Formel (I) enthalten ist, wird mit Hilfe von neuen biologisch aktiven Initiatoren der allgemeinen Formel (VII)

$A$-$L$-$B$-$($-$L$-$A)_y$ (VII)

worin

A der oben definierte biologisch aktive Teil ist,
B ein radikalbildender Teil und
L eine Linkergruppierung und
y die Zahl 0 oder 1 bedeutet,

erhalten.

Als Linkergruppierung L kommen folgende Gruppierungen in Frage:

$-SO_2$-, -COO-, $-SO_2NH$-, -CO-NH-, -NH-CO-O-, -NH-CS-O-, -NH-CO-NH-, -NH-CS-NH-, -O-, -NH-, -S-.

Bevorzugte Linkergruppierungen sind -CO-NH-, -NH-CO-NH-, -COO-, -NH-CO-O. Die Gruppen -CO-NH- und -NH-CO-NH sind besonders bevorzugt.

Die Linkergruppierung L verknüpft den biologisch aktiven Teil und den radikalbildenden Teil B kovalent miteinander.

Falls eine erhöhte Beweglichkeit der Bausteine A und/oder B erwünscht ist, kann durch L auch eine Abstandhalterfunktion ausgeübt werden, wobei L dann aus folgenden Untereinheiten der Formel (VIII) bestehen kann:

$L^1$-R-$L^1$ (VIII)

worin

$L^1$ die bei L angegebenen Bedeutungen hat und

R für $C_1$-$C_{20}$-Alkylen, vorzugsweise $C_3$-$C_{15}$-Alkylen, besonders bevorzugt für $C_5$-$C_{10}$-Alkylen, für $C_6$-$C_{10}$-Arylen-$C_2$-$C_{10}$-Alkylen, vorzugsweise für Phenylen- bzw. Naphthalyen-$C_2$-$C_8$-Alkylen, für $(CH_2$-$CH_2$-$O)_n$ steht, worin

n 1 bis 20, vorzugsweise 3 bis 15 und besonders bevorzugt 3 bis 10, bedeutet.

Als radikalbildender Teil B kommen folgende Verbindungen in Frage:

1) Azostrukturen der allgemeinen Formel (IX)

$R^{11}$-N=N-$R^{12}$ (IX)

worin

$R^{11}$ und $R^{12}$ $C_1$-$C_{20}$-Alkyl, $C_3$-$C_{10}$-Cycloalkyl, $C_7$-$C_{20}$-Aralkyl oder die Gruppe

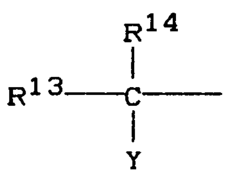

bedeuten, und

Y

CN, $N_3$ oder

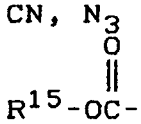 , 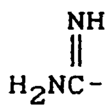 , 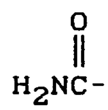 , 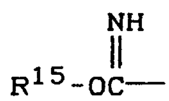

$R^{15}$-O-,

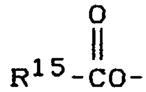 ,

$R^{15}$S-, NCS-, SCN-,

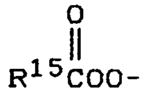 ,

HO- ,

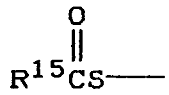 ,

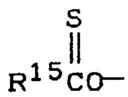 , 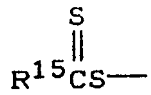 , 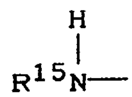 oder 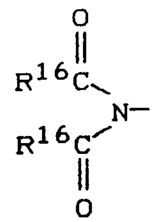

bedeutet,

$R^{13}$, $R^{14}$ und $R^{15}$ unabhängig voneinander $C_1$-$C_{20}$-Alkyl, $C_3$-$C_6$-Cycloalkyl bedeuten oder wenn $R^{13}$ und $R^{14}$ verknüpft sind $C_2$-$C_{30}$-Alkylen bedeuten oder zusätzlich einer der Reste $R^{13}$ oder $R^{14}$, jedoch nicht beide gleichzeitig, Phenyl, Tolyl, Benzyl oder Phenethyl bedeutet,

$R^{16}$ unabhängig $C_1$-$C_6$-Alkyl, $C_3$-$C_6$-Cycloalkyl oder $C_6$-$C_{12}$-Aryl bedeutet;

2) Tetraaryl/alkylethane der allgemeinen Formel (X)

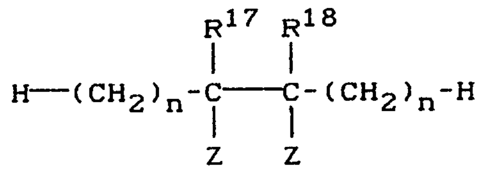

in welcher

Z für Hydroxy, $C_1$-$C_6$-Alkyl oder $C_6$-$C_{20}$, insbesondere für Hydroxy, Methyl, Ethyl, n- oder iso-Propyl, Phenyl oder Naphthyl steht,

$R^{17}$ und $R^{18}$ unabhängig voneinander für $C_1$-$C_6$-Naphthyl, insbesondere für Methyl, Ethyl, n-oder iso-Propyl, Phenyl oder Naphthyl stehen und

n für die Zahlen 1 bis 6, vorzugsweise 1, 2, 3, 4 oder 5, steht;

3) Dinitrile der Formel (XI)

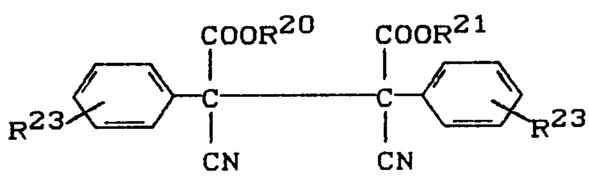 (XI)

in welcher

$R^{20}$, $R^{21}$ und $R^{23}$ unabhängig voneinander für $(CH_2)_m$-H stehen, worin

m für die Zahlen 1 bis 6, vorzugsweise 1, 2, 3, 4 oder 5 steht;

4) Peroxide der allgemeinen Formel (XII)

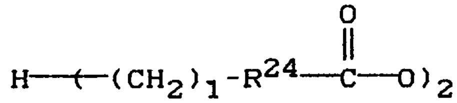 (XII)

in welcher

1 die Zahlen 0 bis 6, vorzugsweise 0, 1, 2, 3 oder 4, insbesondere 0, 1 oder 2, bedeutet und

$R^{24}$ Phenylen, Naphthylen, $C_3$-$C_6$-Alkylen oder $C_3$-$C_6$-Cycloalkylen bedeutet.

Die unter 1) und 4) genannten Strukturen der Bausteine B sind bevorzugt.

Die Verbindungen der Formeln (IX) bis (XII) sind allgemein bekannt (vgl. beispielsweise US-PS 3 956 269, Houben-Weyl, Makromolekulare Stoffe, Teil 1, S. 16-19).

Bevorzugte Azostrukturen der Formel (IX) sind Verbindungen der Formel (IXa)

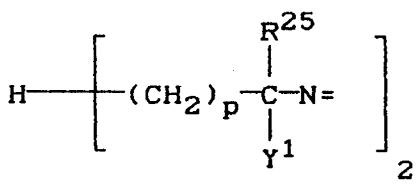

(IXa)

in welcher

p die Zahlen 1 bis 20, vorzugsweise 1 bis 15, insbesondere 2 bis 10, bedeutet,

$Y^1$ für CM, $N_3$, $COOR^{26}$ und

$R^{25}$ und $R^{26}$ unabhängig voneinander $C_1$-$C_6$-Alkyl, insbesondere Methyl, Ethyl, n- oder iso-Propyl, oder $C_3$-$C_6$-Cycloalkyl, insbesondere Cyclopropyl, Cyclopentyl oder Cyclohexyl, bedeuten.

Eine besonders bevorzugte Struktur der unter 1) genannten Bausteine B hat die Formel (IXb)

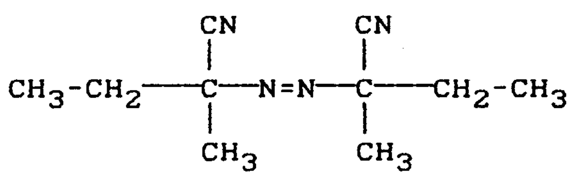

(IXb),

d.h., p bedeutet 2, $R^{25}$ $CH_3$ und $Y^1$ CN.

Eine besonders bevorzugte Struktur der unter 4) genannten Bausteine B entspricht der Formel (XII) mit 1 = O und $R^{24}$ = Phenylen.

Die Strukturen der Formeln (IX) bis (XII), die den radikalbildenden Teil ausmachen, tragen 1 (y in Formel VII = O) oder 2 (y in Formel (VII) = 2) reaktionsfähige Gruppen $X^1$ (vgl. Beschreibung der Formel (XIII)).

In den Azostrukturen der Formel (IX) tragen die Reste $R^{11}$ und $R^{12}$ diese Gruppe(n). In den Strukturen der Formeln (IXa), (IXb), (X) und (XII) sind die endständigen Wasserstoffatome durch diese Gruppe(n) ersetzt. Die Dinitrile der Formel (XI) tragen diese Gruppe(n) symmetrisch um die zentrale Bindung in $R^{20}$ , $R^{21}$ und/oder $R^{23}$.

Im einzelnen seien beispielsweise die folgenden biologisch aktiven Initiatoren der Formel VII aufgezeigt.

Initiator 1:

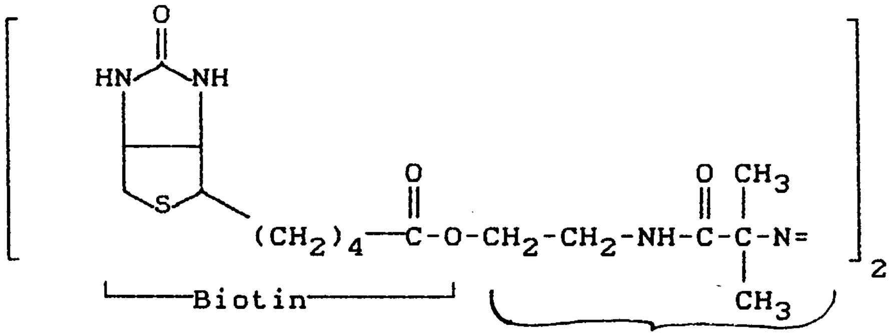

biologisch aktiver Teil A radikalbildender Teil B

Initiator 2:

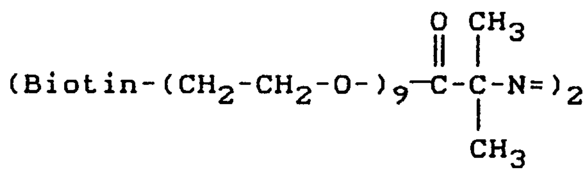

Initiator 3:

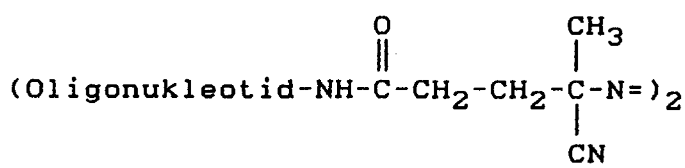

Initiator 4:

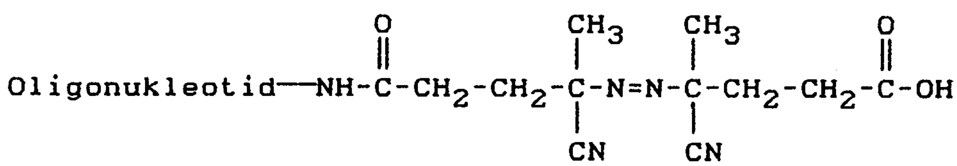

Initiator 5:

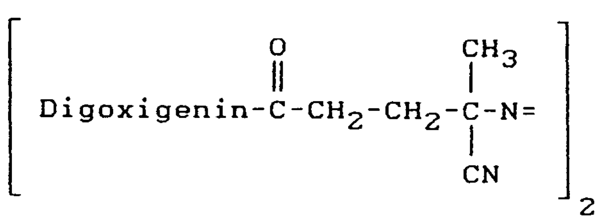

Initiator 6:

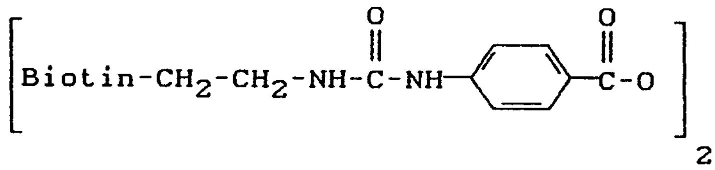

Initiator 7:

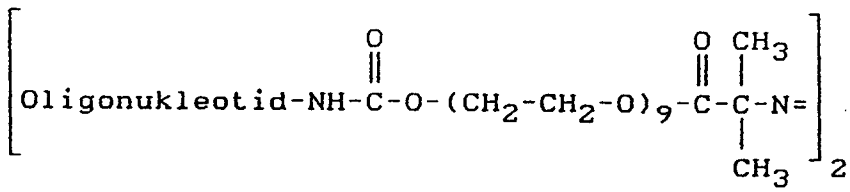

Die biologisch aktiven Radikalketteninitiatoren der allgemeinen Formel (VII)

$A\text{-}L\text{-}B[L—A]_y$ (VII)

werden hergestellt, indem man radikalbildende Verbindungen der allgemeinen Formel (IX)

$X^1—B—(X^1)_y$ (XIII)

wobei

- B wie oben definiert ist und
- $X^1$ NCO, NCS, COCl, COOH, CO-O-NH-Hydroxysuccinimid, OH, $NH_2$, SH, Cl, Br oder I sein kann und
- y 0 oder 1, bevorzugt 1 ist,

mit 1 oder 2 Äquivalenten biologisch aktiven Substanzen A in gegenüber den unter $X^1$ genannten Gruppierungen chemisch inerten Lösungsmitteln, wie beispielsweise Chloraliphaten, Ketone, Nitrile, Sulfoxide, Sulfone u.ä., bei Temperaturen zwischen 0 und 40 °C umsetzt.

Bei Verbindungen der allgemeinen Formel (XIII)

$X^1—B—(X^1)_y$ (XIII)

in denen

- $X^1$ COCl oder $CONHSO_2Cl$ bedeutet,

ist dem Reaktionsgemisch zweckmäßigerweise ein Protonenfänger wie beispielsweise Pyridin oder Triethylamin zuzusetzen, wohingegen, wenn X = COOH, die Umsetzung in Gegenwart von Carbodiimiden, beispielsweise Dicyclohexylcarbodimid, durchgeführt wird.

Als Verbindung der Formel $X^1—B—(X^1)_y$ werden in das erfindungsgemäße Verfahren bevorzugt Verbindungen, in denen $X^1$ = NCO und CO-O-N-Hydroxysuccinimid bedeutet, eingesetzt.

Die Reaktion erfolgt bevorzugt zwischen 0 und 30 °C, besonders bevorzugt zwischen 15 und 25 °C und insbesondere bei ca. 20 °C. Als Lösungsmittel werden bevorzugt Methylenchlorid, Aceton oder Acetonitril eingesetzt.

Die Verbindungen der Formel (XIII) sind allgemein bekannt oder lassen sich nach allgemein bekannten Verfahren herstellen (vgl. z.B. US-PS 4 155 937).

Die Linkergruppierung L wird gebildet durch die Umsetzung von $X^1$ aus der Formel $X^1\text{-}B\text{-}(X^1)_y$ mit der reaktiven Gruppe im biologisch aktiven Baustein A, beispielsweise der Carboxylgruppe in Biotin oder der Aminogruppe im Oligonukleotid.

Die Isolierung der biologisch aktiven Radikalkettenstarter erfolgt nach an sich bekannten Methoden, z.B. nch dem Abfiltrieren von gegebenenfalls gebildeten (ionischen) Nebenprodukten durch Abdampfen des

Lösungsmittels im Hochvakuum, wenn niedrigsiedende Lösungsmittel verwendet werden, oder durch Ausfällung durch Zusatz eines geeigneten Fällungsmittels, wobei das erfindungsgemäße Produkt in der Regel rein anfällt. In den Fällen, in denen die Nebenprodukte nicht flüchtig oder nicht durch Umlösen oder Filtrieren von den erfindungsgemäßen Verbindungen abtrennbar sind, erfolgt die Isolierung der erfindungsgemäßen Verbindungen durch an sich bekannte Methoden der Flüssigchromatographie, beispielsweise Säulenchromatographie oder präparative HPLC (Hochdruckflüssigkeitschromatographie).

Herstellung der biologisch aktiven Polymere der Formel (I):

Durch den (thermisch) initiierten Zerfall des Radikalkettenstarters werden freie Radikale, die den biologisch reaktiven Teil A enthalten, gebildet, die mit den ethylenisch ungesättigten Verbindungen zu neuen freien Radikalen reagieren, welche weitere ethylenisch ungesättigte Monomere addieren, wobei der Abbruch der Wachstumsreaktion durch Kombination zweier wachsender Radikale, durch deren Disproportionierung, Wasserstoffabstraktion oder Kettentransfer (z.B. bei Verwendung von Reglern) erfolgt. Bei Abbruch durch erstere Reaktion entstehen erfindungsgemäße Polymere der Formel A-P-A, wohingegen bei letzteren Reaktionen erfindungsgemäße Polymere der Formel A-P entstehen. Bei mehreren, gleichzeitig auftretenden Abbruchreaktionen bilden sich Gemische der erfindungsgemäßen Polymere aus beiden Formeln. Die Molmassen der erfindungsgemäßen Polymere sind durch an sich bekannte Variationen der experimentellen Details, wie Initiator-, Monomer-, gegebenenfalls Reglerkonzentration, Temperatur und Lösemittel einstellbar.

Durch Verwendung eines mercaptogruppenhaltigen biologisch aktiven Teils, A-SH kann die radikalische Kettenpolymerisation auch unter Verwendung nichtfunktioneller, herkömmlicher Radikalkettenstarter, wie beispielsweise Azoisobutyronitril oder Dibenzoylperoxid initiiert werden, wobei der mercaptogruppen-enthaltende Teil A dann gleichzeitig als Kettenüberträger und Molgewichtsregler fungiert.

$$A\text{-}SH + (M)_n^{\bullet} \longrightarrow A\text{-}S\text{-}(M)_n + H^{\bullet} \xrightarrow{M} A\text{-}P + H\text{-}M^{\bullet}$$

Alternativ können auch sonstige an sich bekannte mono- und bifunktionelle Initiatoren (AnI) für die anionische Polymerisation, verwendet werden, indem der Abbruch der Wachstumsreaktion nach Erreichen der gewünschten Molmasse mit funktionalisierbaren Reagenzien wie beispielsweise $I_2$ oder Br-CN erfolgen kann, woran dann die Verknüpfung mit hydroxy- oder aminofunktionellen Verbindungen, die den biologisch reaktiven Teil A enthalten, in polymeranaloger Weise erfolgt:

$AnI\text{-}(M)_p^{\ominus} + Br\text{-}CN \rightarrow AnI\text{-}(M)_p\text{-}Br + CN^{\ominus}$
$AnI\text{-}(M)_p\text{-}Br + A\text{-}NH_2 \rightarrow P\text{-}A$

Als weiteres Verfahren zur Herstellung erfindungsgemäßer Farbstoffe eignet sich die (lebende) kationische Polymerisation dazu befähigter Monomere unter an sich bekannten Bedingungen, d.h. in Gegenwart kationischer Initiatoren (KatI) wobei die Wachstumsreaktion nach Erreichen der gewünschten Molmasse durch amino-, thio-, hydroxyfunktionelle, den biologisch aktiven Teil enthaltende Verbindungen abgebrochen wird.

$$KatI^{\oplus} + pM \longrightarrow KatI\text{-}(M)_p^{\oplus} \xrightarrow{A\text{-}OH} P\text{-}A + H^{\oplus}$$

Als Modifizierung der lebenden kationischen Polymerisation ist die Inifer-Methode zur Herstellung erfindungsgemäßer Polymerer geeignet, (vergl. Kennedy, J.P.: Polymer J. 12, 609, (1980), wo die Funktion des Initiators und Kettentransferagenz von ein und derselben Verbindung ausgeübt werden.

Die funktionellen Endgruppen können dann in polymeranaloger Weise mit amino- oder thio-funktionellem, biologisch aktivem Teil A enthaltender Verbindung reagiert werden.

Als weiteres Verfahren für die Herstellung erfindungsgemaßer Polymere A-P-A eignen sich Polykondensationsreaktionen, indem man nach an sich bekannten Verfahren zur Polykondensation befähigte Monomere, wie beispielsweise Diole oder Diamine und Bis-Isocyanate oder Bis-Säurechloride, einer Kondensationsreaktion unterwirft, deren Abbruch nach Erreichen der gewünschten Molmasse mit biologisch reaktivem Teil A enthaltenden Verbindungen erfolgt.

Die durch diese Verfahren erhaltenen erfindungsgemäßen Polymere können, falls sie funktionelle Gruppierungen besitzen, in an sich bekannten polymeranalogen Reaktionen weiter umgesetzt werden, um so das gewünschte Eigenschaftsbild zu erreichen, z.B. Wasserlöslichkeit.

Zweckmäßiger und einfacher ist es, die gewünschten Eigenschaften durch die Polymeristion von geeigneten Monomeren einzustellen. Hier sind radikalisch polymerisierbare Monomere in Ihrer Vielseitigkeit der chemischen Eigenschaften den anderen Monomeren überlegen. Daher ist das Verfahren der freien radikalischen Kettenpolymerisation unter Verwendung von Radikalkettenstartern oder Kettenüberträgern, die den biologisch aktiven Teil A enthalten, bevorzugt.

Die erfindungsgemäßen biologisch verknüpfbaren Polymere der Formel (I) lassen sich für immunologische Zwecke wie beispielsweise für Gen-Sonden-Tests oder zur Immobilisierung und Markierung von biologischen Substanzen, wie beispielsweise DNA verwenden.

Die nachfolgenden Beispiele dienen nur der Erläuterung und sind nicht so auszulegen, als ob sie die Art und den Umfang der vorliegenden Anmeldung in irgendeiner Weise einschränken.

## Beispiele

### A) Herstellung von biologisch aktiven Initiatoren

Beispiel 1 (entspricht Initiator 1)

3 mmol Biotin wird mit 1 mmol 2,2'-Azobis[2-methyl-N-(2-hyroxyethyl)-propionamid] und 3 mmol Dicyclohexylcarbondiimid in 20 ml trockenem Dimethylformamid bei Raumtemperatur 18 h unter Reinstickstoff gerührt. Der entstandene Niederschlag wird abfiltriert. Dem Filtrat wird 1 ml konzentrierte wäßrige Ammoniaklösung zugesetzt. Es wird noch eine Stunde bei Raumtemperatur gerührt und nochmals filtriert. Das Filtrat wird auf zerstoßenes Eis gegossen, wobei das erfindungsgemäße Produkt (Initiator 2) ausfällt. Der getrocknete und gewaschene Niederschlag ergibt laut DC-Analyse in Methanol:Chloroform (2:1, $I_2$-Detektion) und $^1$H-NMR das reine erfindungsgemäße Produkt.

Beispiel 2

Herstellung von Oligonukleotid-Initiatoren

1,5 mg (0,26 µmol) des 5'-Aminolink-Oligonukleotids der Sequenz ATCCAGTTGTGTCTTCAAC in Natriumphosphatpuffer (pH = 7,5) werden mit einer Lösung von 6 mg (12,6 µmol) 4,4'-Azobis(4-cyanopentansäure N-Hydroxy-succinimidylester) in Dimethylformamid versetzt. Das Reaktionsgemisch wurde 72 h bei Raumtemperatur gerührt. Das Reaktionsprodukt wird durch präpative HPLC (Hochdruckflüssigkeitschromatographie) an einer RP 18 Säule mit einem in 30 min linear ansteigenden Gradienten von Acetonitril in 0,1 m Triethylammoniumacetat isoliert. Ausbeute 35 % der Theorie.

### B) Biologisch aktive Polymere

Beispiel 3

In 200 ml Methylenchlorid werden 10 g Azobisisobutyronitril (AIBN) sowie 100 g Polyethylenoxid (Molmasse 400), Polyol E 400 zugefügt. Die Lösung wird auf 0 °C abgekühlt und bis zur Sättigung HCl-Gas eingeleitet (ca. 46 g HCl in 3 h). Die resultierende klare Lösung wird über Nacht bei 0 °C gerührt und danach langsam unter Rühren auf 200 g Eis/100 g Wasser gegossen. Nach 2 h Rührzeit werden die beiden Phasen im Scheidetrichter getrennt, die wäßrige Phase wird 3 mal mit Methylenchlorid nachextrahiert und die vereinigten Phasen mit wäßriger $NaHCO_3$ Lösung und Wasser ausgeschüttelt. Die organische Phase wird über $Na_2SO_4$ getrocknet und am Rotationsverdampfer bei Raumtemperatur im Hochvakuum eingedampft. Die Ausbeute beträgt 41,6 g, entsprechend 70,7 % d. Th. Die $^{14}$N-Elementaranalyse ergibt 3,4 % Stickstoff (theor. 3,1 %).

Beispiel 4

In eine Lösung aus 5 g Produkt aus Beispiel 1 in 50 ml Tetrahydrofuran wurde bis zur Sättigung $COCl_2$ eingeleitet. Nach dem Eindampfen bis zur Trockne verbleiben 5,5 g eines viskosen Öls.

Beispiel 5 biotinylierter Starter = Initiator 2

In 15 ml Dimethylsulfoxid (DMSO) werden 1 g $Na_2CO_3$ und 1 g Biotinhydrazid zum Lösen auf 75 °C erwärmt. Nach dem Abkühlen auf 0 °C werden 2,02 g Substanz aus Beispiel 2 zugegeben und über Nacht bei Raumtemperatur nachgerührt. Der Niederschlag wird abfiltriert, das Filtrat am Rotationsverdampfer eingedampft, in Chloroform aufgenommen, mit $NaHCO_3$-Lösung und Wasser ausgeschüttelt und über $Na_2SO_4$ getrocknet. Nach Abdampfen des Lösungsmittels verbleiben 1,26 g eines viskosen Öls.

Beispiel 6

Herstellung eines biologisch aktiven Polymers (Markierungspolymer) aus einem Initiator mit Biotin als biologisch aktiver Endgruppe
In 10 ml trockenem Dimethylsulfoxid werden gelöst:
a) 1,5 g Natrium-p-styrylsulfonat
b) 0,5 g Cumarinfarbstoff 1 und
c) 100 mg des Initiators aus Beispiel 5
Formel des Cumarinfarbstoffs:

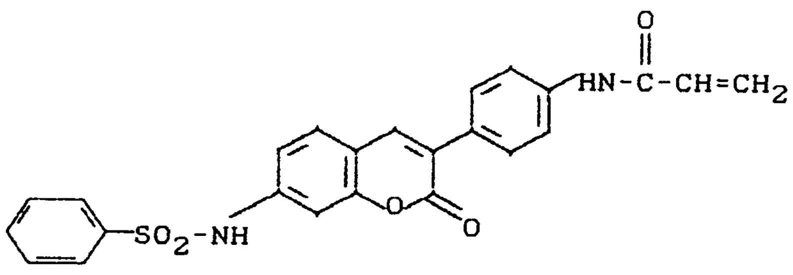

Die Lösung wird mit Reinstickstoff durchgespült, auf 70 °C erhitzt und 16 h bei dieser Temperatur gehalten. Der Rohansatz wird in 200 ml Methanol gefällt, abgesaugt, getrocknet und einer Ultrafiltration (Ausschlußgrenze 10 000 Dalton) unterworfen. Das wasserlösliche, fluoreszierende Polymer hat eine mittlere Molmasse von 110.000 Dalton ($\overline{M}_n$) und kann direkt für Umsetzungen mit Avidin oder Streptavidin eingesetzt werden.

Umsetzung von biotinyliertem Polymer mit Streptavidin:
Eine Lösung aus dem Polymer aus Beispiel 6 (Konz.: 6 mg/ml) wird mit 6 µl Streptavidin (Konz.: 1 mg/ml) versetzt und 8 h lang bei Raumtemperatur geschüttelt.

Das Reaktionsprodukt wird einer SDS-Gelelektrophorese (in Acrylamid) unterworfen. Als Vergleich werden reines Streptavidin und reiner polymerer Farbstoff aufgetragen.

Das Resultat der Elektrophorese ergibt nach Anfärben mit Coumassie-Blue (Farbstoffreagenz auf Proteine), daß das Streptavidin völlig vom Polymerfarbstoff gebunden wurde. Die breite Bande des Reaktionsgemisches zeigt eine grün-blaue Färbung, während keine Bande im Bereich des Streptavidins zu sehen war. Der reine Farbstoff zeigt keinerlei Anfärbung mit Coumassie-Blue. Der reine Farbstoff besitzt aufgrund seiner synthetischen Herstellungsart trotz Ultrafiltration eine Molgewichtsverteilung, die sich auch auf das Reaktionsprodukt, d.h. die Streptavidin-Polymer Kopplungsprodukte überträgt. Diese Kopplungsprodukte können als Marker für biotinylierte Biomoleküle dienen, beispielsweise Antikörper, Nukleinsäure, Proteine.

Beispiel 7

Herstellung eines Markierungspolymeren aus einem Initiator mit Oliginukleotid als biologisch aktiver Endgruppe.

Es wird genauso verfahren, wie in Beispiel 6, statt des Initiators aus Beispiel 5 wird der Initiator aus Beispiel 2 und statt 10 ml Dimethylsulfoxid werden 1,2 ml verwendet:
a) 0,3 g Natriump-p-styrylsulfonat
b) 0,1 g Cumarinfarbstoff 1 und
c) 0,5 mg des Initiators aus Beispiel 2
Das Polymer hat eine mittlere Molmasse von 500 000 ($\overline{M}_n$) und kann direkt in Gensondentests zum Nachweis von DNA oder RNA der Komplimentärsequenz des Oligonukleotids eingesetzt werden.

Beispiel 8

Synthese des Monomer 1 für Polymer 1:
2 g 1,3-Diamino-2-propanol-N,N,N'N'-tetraessigsäure werden in 30 ml Wasser gelöst und mit 2,5 g Triethylamin neutralisiert, das Waasser verdampft und der Rückstand in 50 ml Acetonitril aufgenommen. Dazu werden 0,96 g Methacrylsäureanhydrid und 0,62 g Triethylamin addiert und über 6 h auf ca. 50°C erhitzt. Die Rohlösung wird filtriert, eingeengt und mit 1 n Salzsäure auf pH 3 gestellt. Danach wird die Mischung eingeengt und aus Aceton ausgefällt.

Das Produkt wird im $^1$H-NMR an der charakteristischen Resonanz der Methacrylprotonen bei 5,55 und 6,15 ppm identifiziert. Ausbeute: 56 % der Theorie.

Beispiel 9

Synthese des Monomer 2 für Polymer 2:
Es wurde nach dem gleichen Verfahren gearbeitet wie in Beispiel 8, statt Methacrylsäureanhydrid wurde Itaconsäureanhydrid verwendet.

Beispiel 10

Synthese des Monomer 3 für Polymer 3:
5 g p-Aminosalicylsäure werden in Dimethylacetamid gelöst, 3 g Methacrylsäureanhydrid hinzugefügt und bei 90°C erhitzt. Die Rohlösung wird eingeengt und auf Wasser gegossen. Der Niederschlag wird filtriert, mit Wasser nachgewaschen und getrocknet.

Das Produkt wird im $^1$H-NMR an der charakteristischen Methacrylprotonen bei 5,55 und 6,15 ppm identifiziert. Ausbeute: 65 % der Theorie.

Beispiel 11

Synthese des Monomer 4 für Polymer 4:
Es wurde das gleiche Verfahren angewendet wie unter Beispiel 10, statt Methacrylsäureanhydrid wurde $\alpha,\alpha$-Dimethyl-misopropenylbenzylisocyanat verwendet.

Beispiel 12 bis 15

Herstellung der Polymeren 1-4:
Es wurden bei Polymer 1 und 3 die Polymerisationsbedingungen von Beispiel 6 mit Initiator aus Beispiel 1 und für Polymer 2 und 4 die Bedingungen wie unter Beispiel 7 beschrieben mit Initiator aus Beispiel 2 mit folgenden Monomeren angewendet:

| | Monomer 1 | Monomer 2 | Monomer 3 | Monomer 4 | Kaliumsulfopropylmethacrylat | Molmasse ($\overline{M}n$) |
|---|---|---|---|---|---|---|
| Polymer 1 | 2 g | - | - | - | 2 g | 130.000 |
| Polymer 2 | - | 0,2 g | - | - | 0,2 g | 420.000 |
| Polymer 3 | - | 2 g | 2 g | - | 1 g | 115.000 |
| Polymer 4 | 0,1 g | - | - | 0,2 g | 0,1 g | 380.000 |

Die Polymeren werden einer Ultrafiltration mit Ausschlußgrenze 10.000 Dalton unterworfen und per wäßriger GPC (Gelpermeationschromatographie) auf Molmasse untersucht. Die Polymere können jetzt mit Metallionen beladen
werden und dann zur Umsetzung mit (Strept-)Avidin (Polymere 1 und 3) bzw. zum Nachweis der TA Promoterregion des HIV Gens eingesetzt werden (Polymere 2 und 4).

Biologisch aktive Substanzen, die mit Markierungspolymeren markiert sind

Beispiel 16

Doppelmarkierung der Polymer-Oligonukleotidsonde

Zum Nachweis der Kopplung der Oligonukleotidsonde an das Polymer und zum Vergleich der Nachweisempfindlichkeit der im Polymer gebundenen Cumarin-Fluoreszenzfarbstoffe mit den herkömmlichen $Phosphor^{32}$ ($P^{32}$) oder Digoxigenin-Markierungen wurden eine Doppelmarkierung der Polymer-Oligonukleotidsonde durchgeführt.

Die reaktive 5'-Amino-Oligonukleotidsonde mit der 19 mer Nukleotidsequenz 5'd ATCCAGTTGTGTCTTCAAC aus Beispiel 2 wurde mit alpha $P^{32}$-dCTP unter Verwendung eines Endgruppenmarkierungskits der Firma Boehringer Mannheim am 3'Ende markiert. Die Endgruppenmarkierung wurde alternativ nicht radioaktiv mit Digoxigenin-dUTP vorgenommen. In einem 50 µl Ansatz mit 10 µl Reaktionspuffer (Kaliumkakodylat 1 mol/l; Tris/HCL 125 mmol/l, Rinderserumalbumin 1,25 mg/ml; pH 6,6; 25 °C) 1-2 µg Oligonukleotid, 5 Einheiten Terminale Transferase Kobaltchlorid ($COCl_2$) 2,5 mmol/l und 25 µCi alpha $P^{32}$dCTP werden nach 60 Minuten bei 37 °C ca. 50 % Endmarkierung erreicht.

Die Kopplung an das Polymer wurde wie in Beispiel 7 beschrieben durchgeführt.

Das Polymer wurde in Ethanol gefällt und dann in 1 ml bidestilliertem Wasser gelöst. Durch Gelelektrophorese in 17 %igem Polyacrylamidgel wurde nachgewiesen, daß das Oligonukleotid an das Polymer gebunden ist.

Mit der doppelmarkierten Polymer-Oligonukleotidsonde, die einen Nachweis über die Fluoreszenz des im Polymer gebundenen Cumarin-Fluoreszenzfarbstoffes ermöglicht oder einen Nachweis über das im Oligonukleotid gebundene $P^{32}$ bzw. Digoxigenin wurde eine Slot Hybridisierung wie in dem nachfolgenden Beispiel 17 beschrieben und eine Flüssighybridisierung wie im Beispiel 18 beschrieben durchgeführt.

Beispiel 17

Slot Blot Hybridisierung mit Polymer-Oligonukleotidsonde

Die Hybridisierung wurde nach üblichen Verfahren durchgeführt bei einer Inkubationstemperatur von 40 bis 68 °C.

Je nach Hybridisierungstemperatur wurden unterschiedliche Substanzen zugesetzt. Dextransulfat oder andere Polymere wurden eingesetzt, um die Geschwindigkeit und das Ausmaß der Hybridisierung zu erhöhen. Detergentien und Blockierungs-Reagentien wie Trockenmilch, Denhardt's Lösung, Heparin oder Natriumdodecylsulfat (im folgenden SDS genannt) wurden zugesetzt, um die unspezifische Bindung der DNA an die Membran zu unterdrücken. Denaturierende Agentien wie Harnstoff oder Formamid können eingesetzt werden, um die Schmelztemperatur der Hybride zu reduzieren, so daß niedrigere Hybridierungstemperaturen angewandt werden können. Darüber hinaus können durch Zugabe von heterologer DNA die nicht spezifische Bindung von Gensonden an nicht homologer DNA auf dem Blot reduziert werden.

Zur Vorbereitung der Hybridisierung wurden 50-500 ng der nicht markierten genomischen DNA von Nitrosomonas europeae zunächst 5 Minuten bei 100 °C denaturiert, auf 0 °C abgekühlt und dann auf vorbehandelte Nitrocellulose oder Nylonmembranen mit Hilfe eines Minifold II - Filtrationsgerätes der Firma Schleicher und Schüll aufgetragen und bei 80 °C 2 Stunden fixiert. Die Filter wurden in einer versiegelten Plastik-Folientasche oder Plastikbox mit mindestens 20 ml Hybridisierungslösung pro 100 $cm^2$-Filter bei 68 °C mindestens 1 Stunde hybridisiert.

Die Lösung wurde durch 2,5 ml/100 $cm^2$-Filter Hybridisierungslösung ersetzt, der 100 ng Polymer-Oligonukleotidsonde aus Beispiel 16 zugesetzt wurden. Die Filter wurden mindestens 6 Stunden unter schwachem Schütteln bei 68 °C inkubiert.

Die Filter wurden dann 2 x 5 Minuten bei Zimmertemperatur mit mindestens 50 ml 2 x SSC, 0,1 % SDS pro 100 $cm^2$-Filter und 2 x 15 Minuten bei 68 °C mit 0,1 x SSC, 0,1 % SDS gewaschen.

Die Filter wurden dann direkt zur Detektion der hybridisierten DNA eingesetzt.

Lösungen:

| | |
|---|---|
| 20 x SSC: | 3M NaCl, 0,3 M Na-Citrat pH 7,0 |
| Hybridisierungslösung | 1: 5 x SSC; 0,1 % N-Lauroylsarcosin, Na-Salz; 0,02 % SDS; 0,5 % Blocking Reagenz (Boehringer Mannheim) Lösung bei 50-70 °C lösen |

Andere Hybridisierungslösungen, die ebenfalls für die Slot Blot Hybridisierung eingesetzt werden können, sind z.B:

| | |
|---|---|
| Hybridisierungsmix 2: | 50 % Formamid 7 x SSC; 2 x Denhardt's Lösung (100 x Denhardt's: 2 % Ficoll, 2 % Polyvinylpyrrolidon, 2 % Rinderserumalbumin) 300 µg/ml |

| | |
|---|---|
| | Kalbsthmus-DNA |
| Hybridisierungsmix 3: | 6 x SSC; 10 x Denhardt's-Lösung; |
| | 50 µg Heringssperma DNA, Rinderserumalbumin 0,1 % |
| Hybridisierungsmix 4: | 5 x SSC; PEG; 5 % Trockenmilchpulver |
| | 0,01 M Natriumpyrophosphat; |

Der Nachweis erfolgte über den im Polymer der Oligonukleotidsonde gebundenen Cumarin-Fluoreszenzfarbstoff. Die fluoreszierenden Slot Blots des Filters wurden in einem Shimadzu CS 930 Scanner quantitativ ausgewertet.

Durch die Doppelmarkierung des Oligonukleotids mit $P^{32}$ durch 3'Endgruppenmarkierung mit terminaler Transferase war eine Auswertung des Hybridisierungsversuches auch über Autoradiographie möglich. Das Filter wurde dazu auf einer Glasplatte fixiert und dann ein Röntgenfilm aufgelegt und 2-5 Stunden exponiert. Nach der Entwicklung des Films wurde die Schwärzung des Slot Blots quantitativ mit einem Shimadzu Scanner quantitativ ausgewertet. Alternativ wurden auch andere Nachweismethoden eingesetzt. Mit digoxigenierten Polymer-Oligonukleotid-Sonden wurde ein Farbstoff-Nachweis mit alkalischer Phosphatase konjugierten Antikörpern und Brom-Chlor-Indolylphosphat und Nitroblautetrazolium oder ein Chemilumineszenz-Rad Out mit alkalischer Phosphatase und AMPPD (3-(2'-Spiroadamantan)-4-Methoxy-4-(3''-Phosphoryloxy)-phenyl-1,L-dioxetan) als Substrat durchgeführt.

Durch die Doppelmarkierung war der direkte Vergleich der Sensitivität verschiedener Nachweis-Systeme möglich.

Ergebnis:

Durch die Signalamplifikation der Cumarin-Farbstoffmoleküle im Polymer (ca. 200 Farbstoffmoleküle/Polymer) der Oligonukleotidsonde konnte mit der Detektion von 1-0,1 pg DNA nahezu die gleiche Sensitivität wie mit enzymatischen Nachweismethoden erreicht werden. Durch die Verwendung eines Polymer-Trägers für die Cumarin-Farbstoffmoleküle wird eine um den Faktor 100-1000 höhere Sensitivität erzielt als mit der herkömmlichen Markierung mit Fluoreszenzfarbstoffen über Fluoreszenz-Nukleotidbausteine.

Beispiel 18

Flüssighybridisierung mit der Polymer-Oligonukleotidsonde

Flüssighybridisierungen wurden als Sandwich Hybridisierungen mit Streptavidin gecoateten magnetischen Partikeln der Firam Dynal (Hamburg) zur Separation des Hybridisierungskomplexes durchgeführt.

Die Flüssighybridisierungstests wurden als Sandwich Teste mit 100 ng 5'-biotinylierter Capture Oligonukleotidsonde mit der Nukleotidsequenz 5'd CTGCTCGTAGACAATGCGT, 100 ng Polymer-Oligonukleotidsonde wie im Beispiel 13 (Detector Gensonde) und Nitrosomonas Target-DNA in verschiedenen Konzentrationen (50 ng-1 000 ng) in einem Volumen von 50 µm durchgeführt.

Nach 10 minütigem Erhitzen auf 100 °C und anschließendem Abkühlen auf 0 °C wurden 50 µl 2 x Hybridisierungsmix 1 (Boehringer Mannheim) zugegeben und 1 Stunde bei 68 °C hybridisiert. Die magnetischen Beads wurden mit 1 x Hybridisierungsmix 1 vorbehandelt und nach dem Separieren über einen Magneten die Flüssigkeit abpipettiert und der Hybridisierungsansatz zugegeben und 1/2 Stunde bei Raumtemperatur unter schwacher Bewegung inkubiert. Der gekoppelte Hybridisierungskomplex wurde mit den Beads separiert, die Restflüssigkeit abpipettiert und einmal mit Puffer A (2 x SSC; 0,1 % SDS), dann mit Puffer B (0,1 SSC; 0,1 % SDS) 2 x gewaschen.

Anschließend wurde 500 µl bidest. $H_2O$ zugegeben und die Fluoreszenz der Polymer-Oligonukleotidsonde im Hybridisierungskomplex in einem Fluoreszenzphotometer bei 375 nm Anregung und 495 nm Emission gemessen.

Parallel dazu wurde die Blocking Reaktion und Antikörper-Reaktion zum Nachweis der Hybridisierung über Chemilumineszenz durchgeführt. Die mit DNA beladenen Beads wurden 1 x mit 150 µl Waschpuffer (0,1 M Maleinsäure, 0,1 M NaCl, pH 7,5, 0,3 % Tween 20) behandelt und nach dem Separieren und Abpipettieren des Waschpuffers 400 µl Puffer 2 (0,1 M Maleinsäure; 0,15 M NaCl, pH 7,5; 1 % Blocking Reagenz (Boehringer, Mannheim) zugegeben. Mach 1/2 Stunde Inkubation bei Raumtemperatur wurde separiert, abpipettiert und 100 µl Antikörperkonjugat-Lösung (AK 1:10 000 in Puffer 2) zugegeben und 1/2 Stunde Raumtemperatur inkubiert, dann separiert, abpipettiert und mit 400 µl Waschpuffer behandelt, 2 x 15 Minuten bei schwacher Bewegung. Anschließend wurde separiert und mit 150 µl Puffer 3 (0,1 M Tris/HCl Puffer mit 0,1 M NaCl und 50 mM $MgCl_2$ pH 9,5) behandelt. Es wurde wieder separiert und mit

Detektionslösung mit AMPPD 1:100 im Puffer 3 15 Minuten bei 37°C im Wasserbad inkubiert, dann die Chemilumineszenz im Lumineszenz-Photometer bei 477 nm (Lumacounter von Lumac) gemessen.

Ergebnis

Durch die Signalamplifikation des Cumarinfarbstoffes im Polymer konnte eine deutlich höhere Sensitivität als mit direkter Fluoreszenzmarkierung der DNA erreicht werden. Die Sensitivität liegt um den Faktor 100-1000 höher. Die Detektionsgrenze erreicht mit 1 - 0,1 pg DNA nahezu die Chemilumineszenz-Sensitivität.

Beispiel 19

Hybridisierung der Polymer-Oligonukleotidsonde mit amplifizierter DNA

Durch diese Sensitivitätssteigerung wird der direkte und damit besonders einfache Nachweis über die Fluoreszenz der Polymer-Oligonukleotidsonde eine sehr gute Alternative im Vergleich zu den bekannten Nachweis-Techniken wie Chemilumineszenz, Brom-Chlorindolylphosphat/Nitroblau-Tetrazolium-Farbstoffreaktion und die radioaktive Methoden.

Die Amplifikation der Target-DNA wurde nach der Polymerase Chain Reaktion (EP-A 200 362; 201 184) und alternativ nach der Hairpin Amplifikatons-Methode (EP-A 427 074) durchgeführt.

Zur PCR-Reaktion wurden eingesetzt 2 µg genomische DNA von Nitrosomonas europae, 2 µmol Primer 1 (5'dATCCAGTTGCTTCAAC) und Primer 2 (5'ACTGGCAGGCAGCAG), 2,5 Units Taq-Polymerase von Cetus/Perkin-Elmer sowie jeweils 200 µmol dNTPS in insgesamt 100 µl PCR-Puffer (50 mM KCl, 10 mM Tris/HCl pH 8,3, 1,5 mM $MgCl_2$, und 0,01 % Gelatine). Die Amplifikation wurde in einem PCR-Prozessor der Firma Cetus/Perkin-Elmer durchgeführt. Mit den Ansätzen wurde zunächst eine Initialschmelzung der DNA 2 Minuten, 30 Sek, bei 94°C durchgeführt, dann pro Zyklus 1 Min, bei 94°C die DNA denaturiert, 2 Minuten bei 40-45°C das Primer-Annealing und 3 Minuten bei 72°C die Primer-Extension durchgeführt. Mach 35 Zyklen wurde abschließend eine 20 Minuten-Extension bei 72°C durchgeführt und die Ansätze bei 4°C gekühlt.

Die amplifizierte DNA wurde 5 Minuten bei 100°C denaturiert und dann die Ansätze sofort auf 0°C gekühlt, 200 µl eiskaltes 20xSSC wurden zugegeben und sofort auf Nitrocellulose oder Nylonmembranen mit Hilfe eines Minifold II Filtrationsgerätes der Firma Schleicher und Schüll aufgetragen. Die DNA auf den Filtern wurde 2 Stunden bei 80°C fixiert.

Die Slot Blot Hybridisierung mit der Polymer-Oligonukleotidsonde und Nachweis erfolgte analog wie im Beispiel 18 beschrieben.

Ergebnis

Durch die Kombination der Target-Mukleinsäure-Amplifikation mit der Signalamplifikation des Cumarinfarbstoffes im Polymer wurde eine Sensitivität erreicht, die die Detektion einzelner DNA-Moleküle ermöglicht. Die Auswertung über die Polymer-Fluoreszenz wird damit eine echte Alternative zu der sensitiven Chemilumineszenz-Methode. Im Gegensatz zu der enzymatischen Chemilumeszenzbildung kann die Fluoreszenz im Polymer direkt gemessen werden.

## Patentansprüche

**1.** Biologisch aktive Polymere der allgemeinen Formel (I),

$$P\text{-}(A)_q$$

in welcher
- P eine polymere Komponente
- A ein biologisch aktiver Teil und
- q die Zahl 1 oder 2 bedeutet.

**2.** Biologisch aktive Polymere der Formel (I) gemäß Anspruch 1, worin Acryl-, Methacryl, Vinyl- oder Styryl-Derivate oder Mischungen davon Monomerbausteine der polymeren Komponente sind.

**3.** Biologisch aktive Polymere der Formel (I) gemäß Anspruch 1, worin folgende Monomerbausteine zum Aufbau von P verwendet werden:
Verbindungen der Formel (II):

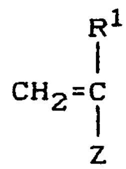

(II)

in welcher

Z für Wasserstoff, $C_1$-$C_{20}$-Alkyl, -CO-$OR^2$, -CO-$NR^3R^4$ oder -$OOCR^5$ steht und

$R^1$, $R^2$, $R^3$, $R^4$ und $R^5$ unabhängig voneinander Wasserstoff oder $C_1$-$C_{20}$-Alkyl bedeuten,

Styrol, α-Methylstyrol oder Verbindungen der Formel (III)

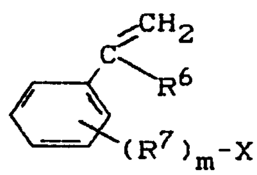

(III)

in der

$R^6$ Wasserstoff oder Methyl,

$R^7$ $CH_2$ oder $SO_2$,

m Null oder 1 und

X Halogen, $SO_2$-$CH_2$-$CH_2$-Halogen, OMe, SO-$CH_3$ oder Methyl, worin Me für ein Äquivalent eines Metalls steht,

bedeuten,

1- und 2-Vinylnaphthalin, 1-Vinylcarbazol und Verbindungen, die zu solchen der Formel (III) analog sind, als aromatischen Grundkörper jedoch Naphthalin oder Carbazol enthalten, sowie sich von aromatischen Aminen, Phenolen, aromatischen Hydroxycarbon-, Hydroxysulfon-, Aminocarbon- und Aminosulfonsäuren ableitenden (Meth)Acrylamide und (Meth)Acrylate der Formel (IV)

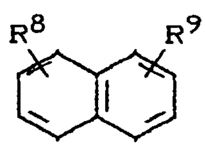

(IV)

in der

$R^8$ für Wasserstoff, $SO_3H$, COOH, $SO_3Me$ oder COOMe steht, wobei Me ein Äquivalent eines Metalls ist, bedeutet und

$R^9$ für

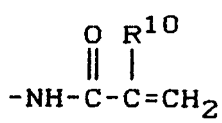 oder 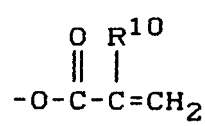

mit $R^{10}$ = Wasserstoff oder Methyl

steht.

4. Biologisch aktive Polymere gemäß Anspruch 1, worin A Biotin, Digoxigenin, Digoxin, Digitoxigenin oder Oligonukleotide aus 1 bis 80 Nukleotidbausteinen ist.

5. Biologisch aktive Polymere gemäß Anspruch 1 bis 3, dadurch gekennzeichnet, daß die Monomerbausteine chelatisierende und/oder wasserlöslichkeitsvermittlende und/oder Farbstoffgruppen enthalten.

6. Biologisch aktive Polymere gemäß Anspruch 5, dadurch gekennzeichnet, daß 10-90 Gew.-% wasserlöslichkeitsvermittelnde und/oder chelatisierende Gruppen bezogen auf das Gesamtpolymer (= 100 Gew.-%) im Polymer enthalten sind.

7. Biologisch aktive Polymere gemäß Anspruch 5, dadurch gekennzeichnet, daß der Anteil der Farbstoffgruppen 1 bis 99 Gew.-% bezogen auf das Gesamtpolymer ist.

8. Verwendung von biologisch aktiven Polymeren gemäß Anspruch 1 für immunologische Zwecke und zur Markierung von biologischen Substanzen.